# EUROPÄISCHE PATENTANMELDUNG

(11) **EP 1 229 326 A2**
(43) Veröffentlichungstag der Anmeldung: **07.08.2002**
(21) Anmeldenummer: 02001693.7
(22) Anmeldetag: 24.01.2002
(51) Int. Cl.: G01N 33/38

(54) **Verfahren zum Messen des Luftporenanteils von Frischbeton**

(30) Priorität: 06.02.2001 DE 10105198; 14.03.2001 DE 20104348 U
(71) Anmelder: Gesellschaft neue Technologie-Verwertung, GntV GmbH, 76571 Gaggenau (DE)
(72) Erfinder: Vollbrecht, Wolfgang, 76571 Gaggenau (DE)
(74) Vertreter: Geitz & Truckenmüller

(57) **Zusammenfassung**

Bei dem Verfahren wird ein gas- und wasserdicht verschlossener Probenbehälter mit einer darin aufgenommenen verdichteten Frischbetonprobe verwogen und im Anschluß daran über der Betonprobe vollständig mit Wasser blasenfrei aufgefüllt, sowie anschließend erneut verwogen. Danach wird der Probenbehälter und damit die in diesem aufgenommene Betonprobe bzw. die über dieser befindliche Flüssigkeit mit unter einem vorbestimmten Druck stehenden Hydraulikmittel beaufschlagt, dann der Probenbehälter hermetisch abgesperrt und von der Druckmittelquelle abgekuppelt sowie erneut verwogen. Die erste Wiegung dient der Ermittlung des Gewichts der Betonprobe und durch Differenzbildung zwischen der ersten und zweiten Wiegung ergibt sich das Gewicht der Flüssigkeitsfüllung über der Probe. Die dritte Wiegung abzüglich der zweiten Wiegung ergibt dann das Gewicht der unter Druckbeaufschlagung in den Probenbehälter eingedrungenen Flüssigkeit als Maß für den Porenanteil im Beton.

## Beschreibung

Die Erfindung bezieht sich auf ein Verfahren zum Messen des Luftporenanteils von Frischbeton, bei dem der Frischbeton in einen gas- und flüssigkeitsdicht verschließbaren Probenbehälter eingebracht und verdichtet, dann der Probenbehälter verschlossen und über der Betonprobe vollständig mit Hydraulikmittel blasenfrei aufgefüllt wird. Ferner bezieht sich die Erfindung auf eine Vorrichtung zum Messen des Luftporenanteils von Frischbeton, die einen topfartig ausgebildeten Probenbehälter mit einem nach oben offen, über die Höhe gleichbleibenden Querschnitt aufweisenden sowie mittels eines Deckels gas- und flüssigkeitsdicht verschließbaren Aufnahmeraum für eine Betonprobe besitzt, der bei gleichzeitiger Entlüftung über wenigstens ein absperrbares Entlüftungsventil mit Wasser oder einer anderen Flüssigkeit befüllbar ist.

Für die Beurteilung eines Betons ist es wichtig, den Luftporengehalt des Frischbetons zu kennen. Hierfür sind bereits eine Vorrichtung und ein damit durchführbares Verfahren bekannt.

Die vorbekannte Vorrichtung besitzt einen mittels eines Deckels gas- und flüssigkeitsdicht abschließbaren Probenbehälter, in den zum Zwecke der Luftporenbestimmung Frischbeton eingebracht und verdichtet wird. Danach wird der Probenbehälter mittels des Deckels verschlossen und der von der Betonprobe nicht ausgefüllte Raum des Behälters bei gleichzeitiger Entlüftung vollständig mit Wasser blasenfrei aufgefüllt. Der mit Wasser verfüllbare Behälterraum über der Betonprobe steht über ein Ventil mit einer Vorkammer in Verbindung, die mit Druckluft einer definierten Spannung beaufschlagbar ist. Durch das Öffnen eines Ventils zwischen Vorkammer und dem Probenbehälter wirkt die vorgespannte Druckluft aus der Vorkammer auf die Wasserfüllung über der Betonprobe mit der Folge, daß ein gewisser Druckausgleich stattfindet und infolge der dadurch bedingten Druckbeaufschlagung der Wasserfüllung im Probenbehälter Wasser in die Betonporen gepreßt und die in diesen befindliche Luft zusammengedrückt wird. Die bei dem Druckausgleich eintretende Druckabsenkung in der Vorkammer erfaßt ein an diese angeschlossenes Manometer als Maß für den Luftporenanteil in der Betonprobe. Die Manometeranzeige ist so eingerichtet, daß nicht der sich nach dem Druckausgleich in der Vorkammer einstellende Druck angezeigt wird, sondern der Luftporenanteil der Betonprobe in Volumenprozent.

Das mit der vorbekannten Vorrichtung durchführbare Verfahren zum Messen des Luftporenanteils in verdichtetem Frischbeton ist ungenau und genügt den für eine verläßliche Qualitätsbeurteilung eines Betons an die Luftporenbestimmung zu stellenden Anforderungen nicht hinlänglich.

Insbesondere unzulänglich ist die bei derartigen Vorrichtungen erforderliche empirische Festlegung der Manometeranzeige, weil die Berechnung wegen unterschiedlicher Volumina und des bei dem jeweiligen Volumen sich einstellenden verdichteten Luftanteils sehr schwierig ist.

Ein weiteres Problem besteht in der mit einem steigenden Luftporenvolumen zunehmenden Ungenauigkeit der Messung. So weist die Manometerskala bei einem Luftporenanteil von ca. 1,0 Prozent eine Anzeigeungenauigkeit von etwa 0,1 Prozent auf, bei einem Porenanteil von ca. 5,0 Prozent eine Ungenauigkeit von etwa 0,2 Prozent, jedoch bei einem Porenanteil von 10 Prozent bereits Abweichungen von etwa 0,5 Prozent. Ferner hat sich gezeigt, daß die Einstellung des Druckes in der Vorkammer schon allein durch temperaturbedingte Schwankungen der Luftdichte nur unzureichend genau möglich ist. Der in aller Regel auf 1,0 Bar Überdruck eingestellte Vordruck in der Vorkammer fällt bei einem Porenanteil von 5,0 Prozent auf etwa 0,5 Bar ab. Je geringer der eingestellte Vordruck ist, um so schwieriger wird es, einen Druckausgleich herzustellen. Mit fallendem Vordruck wird jedoch die Anzeige immer ungenauer.

Durch die Erfindung sollen daher ein gattungsgemäßes Verfahren und eine gattungsgemäße Vorrichtung geschaffen werden, die eine präzisere Messung des Porenanteils von verdichtetem Frischbeton als das Verfahren und die vorbekannte Vorrichtung nach dem Stande der Technik ermöglichen.

In verfahrenstechnischer Hinsicht ist die Erfindungsaufgabe dadurch gelöst, daß bei dem Verfahren nach dem Oberbegriff des Patentanspruchs 6 der Probenbehälter mit der verdichteten Betonprobe vor und nach dem Auffüllen mit Hydraulikmittel jeweils verwogen und das Gewicht der blasenfreien Hydraulikmittelverfüllung durch Differenzbildung der beiden vorausgegangenen Wiegungen bestimmt wird, daß danach der Probenbehälter an eine Druckmittelquelle angekuppelt und mit unter einer vorbestimmten Spannung stehendem Hydraulikdruckmittel beaufschlagt, dann hermetisch gegenüber der Druckmittelquelle abgesperrt und von dieser abgekuppelt sowie erneut verwogen wird, und daß anschließend als Maß für das Porenvolumen des Frischbetons das Gewicht des in die Betonporen eingedrungenen Hydraulikmittels durch Differenzbildung zwischen der dritten und zweiten Wiegung bestimmt wird.

Dieses Verfahren hat sich als einfach handhabbar und äußerst präzise erwiesen, weil das durch Differenzbildung zwischen der dritten und zweiten Wiegung bestimmte Gewicht gleich dem bei der Druckbeaufschlagung in die Betonporen eingedrungenen Wassers ist und mithin dieses Gewicht ein präzises Maß für den Porenanteil im Beton gibt.

Eine zweckmäßige Weiterbildung dieses Verfahrens sieht vor, daß die blasenfreie Verfüllung des Probenbehälters über der in diesem aufgenommenen Betonprobe bei Atmosphärendruck, also etwa 1,0 Bar erfolgt.

Die Präzision der Bestimmung des Porenanteils im Beton ist abhängig vom Druckniveau des zur Beaufschlagung der zuvor über der Probe in den Behälter eingebrachten Hydraulikmittelverfüllung eingesetzten Hydraulikdruckmittels. Eine den Erfordernissen genügende Präzision wird erreicht, wenn das zur Druckbeaufschlagung eingesetzte Hydraulikmittel einen Druck von etwa 3,5 Bar aufweist.

Grundsätzlich können bei dem erfindungsgemäßen Verfahren hydraulische Flüssigkeiten beliebiger Art verwendet werden, sofern diese eine das Eindringen in die Betonporen begünstigende Viskosität haben. Als besonders einfach und darüber hinaus kostengünstig hat sich die Verwendung von Wasser als Hydraulikmittel erwiesen.

In vorrichtungstechnischer Hinsicht ist die Erfindungsaufgabe dadurch gelöst, daß bei der im Oberbegriff des Patentanspruchs 1 angegebenen Vorrichtung an einen Einlaß des Probenbehälters, der mit einem Absperrventil ausgerüstet ist, ein Hydraulikdruckmittelanschluß ankuppelbar und der Probenbehälter mit Hydraulikmittel einer definierten Spannung beaufschlagbar ist.

Im Unterschied zum Stande der Technik, bei dem die Wasserfüllung über der verdichteten Betonprobe mit Druckluft aus einer Vorkammer beaufschlagt wird, ermöglicht es die erfindungsgemäß ausgebildete Vorrichtung, die Hydraulikmittelfüllung über der Betonprobe mit inkompressiblem Hydraulikmittel eines vorbestimmten Druckes zu beaufschlagen. Bei der Flüssigkeitsverfüllung über der Betonprobe und dem unter Druck stehenden Hydraulikmittel kann es sich vorzugsweise um Wasser handeln.

Der Vorteil der erfindungsgemäßen Vorrichtung besteht darin, daß die in den Poren der Frischbetonprobe enthaltene Luft unabhängig vom Porengehalt einem konstanten Druck ausgesetzt und nach klar definierten physikalischen Gesetzmäßigkeiten zusammengepreßt wird. Das Porenvolumen verringert sich in dem Maße, indem Wasser in den Probenbehälter gedrückt wird, wodurch das Gewicht des Probenbehälters ansteigt. Aus dieser Gewichtszunahme ist das Volumen der in den Poren verdrängten Luft und damit das Eindringwasser bestimmbar, dessen Volumen dem Luftporenanteil im Beton entspricht.

Gemäß einer sinnvollen Weiterbildung der Erfindung ist bei der Vorrichtung der Hydraulikmittelanschluß als mit einer Druckmittelquelle verbindbare und mittels einer Schnellkupplung an den Einlaß des Probenbehälters ankuppelbare Armatur ausgebildet. Dies ermöglicht ein schnelles An- und Abkuppeln der Armatur und erleichtert das jeweilige Verwiegen des Probenbehälters.

Zweckmäßigerweise ist die Armatur mit einem Absperrventil sowie einem zwischen letzterem und der Schnellkupplung angeordneten Druckminderventil ausgerüstet, das es ermöglicht, den Druck zur Beaufschlagung der Hydraulikmittelverfüllung über der Probe präzise einzustellen.

Eine weitere Vereinfachung in der Handhabung der Vorrichtung ist gegeben, wenn die Armatur angrenzend an die Schnellkupplung mit einem Ablaßventil ausgerüstet ist, das nach der Druckbeaufschlagung des Probenbehälters und dem Absperren der Armatur von der Druckmittelquelle eine Entspannung des dann im Druckbehälter herrschenden Druckes ermöglicht. Danach kann mittels der Schnellkupplung die Armatur in einfacher Weise von dem Probenbehälter getrennt werden.

Eine abermalige wichtige Weiterbildung der Erfindung sieht vor, daß der Probenbehälter mittels eines auf diesen aufsetzbaren Deckels verschließbar ist und daß der mit einem Absperrventil ausgerüstete Behälter-Einlaß und das Entlüftungsventil am Deckel angeordnet sind.

Bei einer anderen Ausgestaltung der erfindungsgemäßen Vorrichtung ist stirnendig auf der Offenseite des Probenbehälters ein abnehmbarer Rahmen, der eine mit dem Querschnitt des Behälter-Aufnahmeraums deckungsgleiche Ausnehmung aufweist, unter Zwischenlage einer umlaufenden Dichtung aufgenommen sowie auf den Rahmen ein Deckel mit einer sich um die Ausnehmung des Rahmens herumerstreckenden Dichtung und einem auf der zum Rahmen hinweisenden Seite innerhalb der von der Dichtung umschlossenen Fläche ausmündenden, mit einem absperrbaren Ventil versehenen Einlaß für Hydraulikmittel aufsetzbar, der mittels einer lösbaren Spanneinrichtung mit dem auf dem Rand des Probenbehälters aufsitzenden Rahmen verspannbar ist.

Charakteristisch für diese Ausgestaltung der erfindungsgemäßen Vorrichtung ist der abnehmbare Rahmen, der auf der Offenseite des Probenbehälters aufsitzt und eine mit dem Querschnitt des Aufnahmeraums des Probenbehälters deckungsgleiche Ausnehmung aufweist.

Bei bestimmungsgemäßer Verwendung der Vorrichtung wird bei abgenommenem Deckel Frischbeton in den Aufnahmeraum des Probenbehälters eingefüllt und verdichtet. Die Probenfüllung kann dabei durchaus bis in den Bereich der Ausnehmung in den aufgesetzten Rahmen hineinragen. Das Verdichten der Betonprobe erfolgt in bekannter Weise z.B. mittels eines Rütteltischs.

Wie beim Stande der Technik wird der Probenbehälter nach dem Einfüllen und Verdichten der Betonprobe mittels eines die Ausnehmung in dem aufgesetzten Rahmen überdeckenden Deckels gas- und flüssigkeitsdicht verschlossen. Danach wird die Vorrichtung mit dem verschlossenen Probenbehälter und der darin aufgenommenen Frischbetonprobe verwogen.

Der nächste Schritt zur Bestimmung des Porenanteils des Frischbetons besteht darin, daß der Hohlraum zwischen der eingefüllten Betonprobe und dem den Aufnahmeraum abschließenden Deckel bei geöffnetem Entlüftungsventil über den Einlaß und das dann in Offenstellung stehende Einlaßventil mit Hydraulikmittel blasenfrei vollständig verfüllt wird. In aller Regel kommt als Hydraulikmittel Wasser in Betracht. Nach dem Schließen des Entlüftungsventils und des Einlaßventils wird durch erneutes Verwiegen das Gewicht der über der Betonprobe eingebrachten Hydraulikmittelfüllung bestimmt.

Nach dem zweiten Verwiegen erfolgt eine Hydraulikmittelbeaufschlagung des über der Probe eingefüllten Hydraulikmittels und damit der in dem Aufnahmeraum enthaltenen Frischbetonprobe bei einem vorbestimmten Druck von beispielsweise 3,5 Bar. Auch als Hydraulikdruckmittel wird vorzugsweise Wasser eingesetzt. Infolge dieser Druckmittelbeaufschlagung erfährt das in den Poren der Betonprobe enthaltene Luftvolumen eine Zusammendrückung und entsprechend mehr Wasser dringt in den Behälter, worauf die Vorrichtung erneut verwogen wird.

Die erste Wiegung führt zur Ermittlung des Gewichtes der Betonprobe, und die zweite Wiegung zur Ermittlung der über der Probe aufgenommenen Hydraulikmittelfüllung, indem die Differenz zwischen den beiden Wiegungen gebildet wird. Die dritte Wiegung führt zur Ermittlung des infolge Druckbeaufschlagung in die Poren eingedrungenen Hydraulikmittels, indem die Differenz zwischen der dritten und zweiten Wiegung gebildet wird.

Da die erfindungsgemäße Vorrichtung nicht, wie die Vorrichtung nach dem Stande der Technik, mit einem in einer Vorkammer gespeicherten gasförmigen Medium als Druckbeaufschlagung arbeitet, sondern mit einem in dem hier in Betracht kommenden Druckbereich praktisch inkompressiblen Druckmedium, ist eine gegenüber dem Stande der Technik weit präzisere Bestimmung des Luftporenanteils von Frischbetonproben möglich.

Als vorteilhaft hat sich auch erwiesen, wenn der oberseitig auf dem Probenbehälter aufsitzende Rahmen auf der zum Probebehälter hinweisenden Seite eine die mit dem Aufnahmeraum querschnittsgleiche Ausnehmung umgebende Einsenkung aufweist, die der Außenkontur des Probenbehälters im Bereich der Behälteroffenseite angepaßt ist und in die der obere Rand der Behälterwand eingreift. Durch diese Maßnahme ist eine zentrische Aufnahme des Rahmens auf dem Probenbehälter gewährleistet und sichergestellt, daß die Ausnehmung des Rahmens sich in deckungsgleicher Lage mit dem Aufnahmeraum des Probenbehälters befindet.

Bei der zuletzt genannten Ausbildungsvariante ist es sinnvoll, wenn die mit der Oberkante der Behälterwand zusammenwirkende Dichtung des auf der Behälteroffenseite aufsitzenden Rahmens als in einer im Bereich der Einsenkung umlaufenden Nut aufgenommene O-Ringdichtung ausgebildet ist.

Auch bei der im aufgesetzten Zustand des Deckels zwischen diesem und dem Rahmen wirkenden Dichtung kann es sich zweckmäßigerweise um eine in einer im Deckel umlaufende Nut aufgenommene O-Ringdichtung handeln.

Als Einrichtung zum Verspannen des Deckels mit dem Rahmen, und gegebenenfalls auch des Rahmens mit dem Probenbehälter, dient zweckmäßigerweise eine in einer Spindelmutter oder Gewindebohrung geführte Gewindespindel, die zentrisch am Deckel angreift und im angezogenen Zustand die O-Ringdichtung an den Rahmen und gegebenenfalls die O-Ringdichtung des Rahmens an die Oberkante der Behälter-Wandung anpreßt.

Gemäß einer abermals wichtigen Ausgestaltung der Erfindung ist der Probenbehälter auf einer Grundplatte eines Säulengestells zwischen wenigstens zwei von der Grundplatte nach oben vorstehenden Säulen aufgenommen und über dem Probenbehälter erstreckt sich beabstandet von diesem eine an den Säulen festgelegte Spannbrücke, mit der die Gewindespindel führenden Spindelmutter oder Gewindebohrung. Die als Quertraverse ausgebildete Spannbrücke kann in einfacher Weise mittels Spannschrauben auf den dann oberseitige Gewindeabschnitte aufweisenden Säulen lagefest positioniert sein.

Eine besonders einfache Art der Festlegung des auf der Offenseite des Probenbehälters aufsitzenden Rahmens ergibt sich, wenn der Rahmen seitlich vom Probenbehälter vorstehende Abschnitte mit Lochungen aufweist und auf den Säulen aufgenommen sowie mit dem Probenbehälter verspannt ist. Dies kann in einfacher Weise mittels zwischen dem Rahmen und der Spannbrücke auf den Säulen aufgenommener Klemmhülsen verwirklicht sein.

Eine andere zweckmäßige Weiterbildung der Erfindung sieht vor, daß der Aufnahmeraum des Probenbehälters quadratisch ausgebildet ist und eine Höhe gleich einer Kantenlänge des seinen Querschnitt bildenden Quadrats aufweist. Bei einem so ausgebildeten Aufnahmeraum des Probenbehälters gelingt einerseits die Bestimmung des Porenanteils in Beton auf einfache Weise, darüber hinaus aber auch die Herstellung von Probewürfeln für nachfolgende Druckmessungen.

Derartige Probewürfel haben in der Regel eine Kantenlänge von 150 mm. Demgemäß bietet sich an, die Kantenlänge des den Querschnitt des Aufnahmeraums bildenden Quadrats und die Höhe des Aufnahmeraums entsprechend zu bemessen.

Wenn neben der Bestimmung des Porenanteils mittels der Vorrichtung Probewürfel hergestellt werden sollen, muß beim Einfüllen der Frischbetonprobe darauf geachtet werden, daß die Probe über die Oberkante des Probenbehälters hinaus bis in die mit dem Probenbehälter querschnittsgleiche Ausnehmung des aufgesetzten Rahmens hineinragt. Nach der Bestimmung des Porenanteils sind der Behälter-Deckel und der auf dem Probenbehälter aufsitzende Rahmen zu entfernen, worauf der nach oben über den Rand des Probenbehälters vorstehende Beton mittels eines Lineals über den Rand des Probenbehälters abzustreichen ist. Die im Aufnahmeraum enthaltene Betonprobe weist dann präzise die Abmessungen eines Probewürfels auf.

Eine andere vorteilhafte Weiterbildung sieht vor, daß ein den Aufnahmeraum unterseitig abschließender Behälterboden von wenigstens einer mittels eines entfernbaren Verschlußorgans verschlossenen Ausnehmung durchbrochen ist. Diese Ausnehmung im Behälterboden kann sich zweckmäßigerweise zum Aufnahmeraum des Probenbehälters hin konisch verengen und mittels eines konisch angepaßten Stopfens als Verschlußorgan verschließbar sein.

Eine derartige Ausnehmung im Behälterboden ermöglicht nach dem Entfernen des Verschlußorgans eine unterseitige Beaufschlagung der im Aufnahmeraum enthaltenen Betonprobe. Logischerweise muß die Betonprobe beim Ausformen etwa infolge unterseitiger Wasserdruckbeaufschlagung hinreichend abgebunden und gestaltfest sein.

Zwei Ausführungsformen der erfindungsgemäßen Vorrichtung sollen anhand der beigefügten Zeichnung nachstehend erläutert werden. In schematischen Ansichten zeigen:
- Fig. 1: in schematischer Darstellung eine erste Ausführungsform der erfindungsgemäßen Vorrichtung zum Messen des Luftporenanteils von Frischbeton,
- Fig. 2: eine zweite Ausführungsform der Vorrichtung zum Bestimmen des Luftporenanteils von Frischbeton in einer teilweise geschnitten dargestellten Vorderansicht,
- Fig. 3: die Vorrichtung in einer Seitenansicht zu Fig. 2,
- Fig. 4: einen Probenbehälter der Vorrichtung gemäß Fig. 2 mit einem auf diesem aufgenommenen Rahmen und einem auf letzteren aufgesetzten Deckel in einer der Schnittlinie IV-IV in Fig. 2 entsprechenden Schnittansicht,
- Fig. 5: eine Unteransicht des auf dem Probenbehälter aufsitzenden Rahmens,
- Fig. 6: eine unterseitige Ansicht des auf dem Rahmen aufgenommenen Deckels,
- Fig. 7: den Deckel in einer der Schnittlinie VII-VII in Fig. 6 entsprechenden Schnittansicht,
- Fig. 8: eine Draufsicht auf eine Spannbrücke.
- Fig. 9: die Spannbrücke in einer Vorderansicht zu Fig. 8.

Die erste Ausführungsform der Vorrichtung 10 umfaßt einen topfartig ausgebildeten Probenbehälter 11 mit einem Aufnahmeraum 12, der unterseitig von einem Boden 13 abgeschlossen und von einer zylindrischen Behälterwand 14 umgeben ist. Die Offenseite des Probenbehälters 11 weist nach oben und ist mittels eines auf diese aufsetzbaren Deckels 15 gas- und flüssigkeitsdicht verschließbar.

Nach der vom Behälter-Innenraum 12 wegweisenden Seite erstreckt sich vom Deckel 15 ein Entlüftungsstutzen 16 mit einem manuell zwischen einer Schließ- und einer Öffnungsstellung betätigbaren Entlüftungsventil 17 fort. Ferner ist der Deckel 15 mit einem Einlaßstutzen 18 versehen, der sich ebenfalls nach der vom Behälter-Innenraum 12 wegweisenden Seite vom Deckel 15 forterstreckt und mit einem ebenfalls manuell zwischen einer Öffnungs- und Schließposition bewegbaren Absperrventil 19 ausgerüstet ist.

Die Vorrichtung 10 umfaßt weiter eine Armatur 20 als Druckwasseranschluß, die in hier nicht weiter interessierender Weise an einen mit einer nicht dargestellten Druckwasserquelle verbundenen Druckwasserschlauch 21 anschließbar ist. Die Armatur 20 ist mit einem Absperrventil 22, einem Druckminderventil 23 und einem Ablaßventil 24 sowie mit Schnellkupplungsmitteln 25 zum Ankuppeln an den sich vom Deckel 15 forterstreckenden Einlaßstutzen 18 ausgerüstet. Der Einlaßstutzen 18 besitzt an seinem vom Deckel 15 entfernten Ende zu den Kupplungsmitteln 25 der Armatur 20 passende Kupplungsmittel 26, etwa einen Kupplungsstecker, in welchem Falle die Armatur 20 mit einer zu diesem Kupplungsstecker passenden Kupplungsmuffe ausgerüstet ist. Das Absperrventil 22 der Armatur 20 ist druckwasserschlauchseitig angeordnet, das Ablaßventil 24 hingegen angrenzend an die Schnellkupplungsmittel 25 in der Nähe des anderen Armaturenendes. Das Druckminderventil 23 befindet sich zwischen dem Absperrventil 22 und dem Ablaßventil 24.

Bei bestimmungsgemäßer Verwendung der Vorrichtung wird zunächst bei abgenommenem Deckel 15 in den Behälter-Innenraum 12 des Probenbehälters 11 Frischbeton eingefüllt und dann beispielsweise durch Rütteln auf einem Rütteltisch verdichtet. Danach wird der Deckel 15 auf die Offenseite des Probenbehälters 11 gas- und flüssigkeitsdicht aufgesetzt und in seiner Schließlage fixiert. Anschließend wird der verschlossene Probentopf mit der in diesem aufgenommenen verdichteten Betonprobe verwogen.

Als nächster Schritt erfolgt die vollständige Verfüllung des zwischen dem aufgesetzten Deckel und der Betonprobe vorhandenen Hohlraums bei gleichzeitiger Entlüftung über das dann in Offenstellung stehende Entlüftungsventil 17 mit blasenfreiem Wasser bei Atmosphärendruck. Danach werden das Entlüftungsventil 17 und das Einlaßventil des Einlaßstutzens geschlossen und der Probenbehälter wird zur Bestimmung der über der Betonprobe aufgenommenen Wasserfüllung erneut verwogen. Die Wasserverfüllung ergibt sich aus der Differenz der beiden Wiegeergebnisse.

Mittels der dem Einlaßstutzen 18 und der Armatur 20 zugeordneten Schnellkupplungsmittel 25, 26 wird dann bei geschlossenem Absperrventil 22 die Armatur 20 an den Einlaßstutzen 18 angekuppelt. Anschließend werden das Einlaßventil 19 des Einlaßstutzens 18 und das Absperrventil 22 der Armatur 20 geöffnet. Dadurch wird die im Probenbehälter aufgenommene Betonprobe bzw. die über dieser aufgenommene blasenfreie Wasserfüllung über den Druckminderer 23 mit einem Wasserdruck von ca. 3,5 Bar beaufschlagt. Angesichts dieser Druckbeaufschlagung dringt in die Betonporen unter Zusammendrückung der in diesen befindlichen Luft Wasser ein. Danach wird das Einlaßventil 19 des Einlaßstutzens 18 in seine Schließstellung und nach dem Schließen des Absperrventils 22 der Armatur 20 deren Ablaßventil 24 geöffnet. Danach erfolgt das Abkuppeln der Armatur 20 vom Einlaßstutzen und anschließend wird der Probenbehälter 11 erneut verwogen, um die bei der Druckbeaufschlagung in den Probenbehälter nachgeströmte Wassermenge zu bestimmen. Dies geschieht durch Differenzbildung der Wiegeergebnisse aus den beiden letztgenannten Wiegevorgängen. Die so bestimmte Wassermenge ist ein Maß für das Volumen der in der Betonprobe enthaltenen Poren.

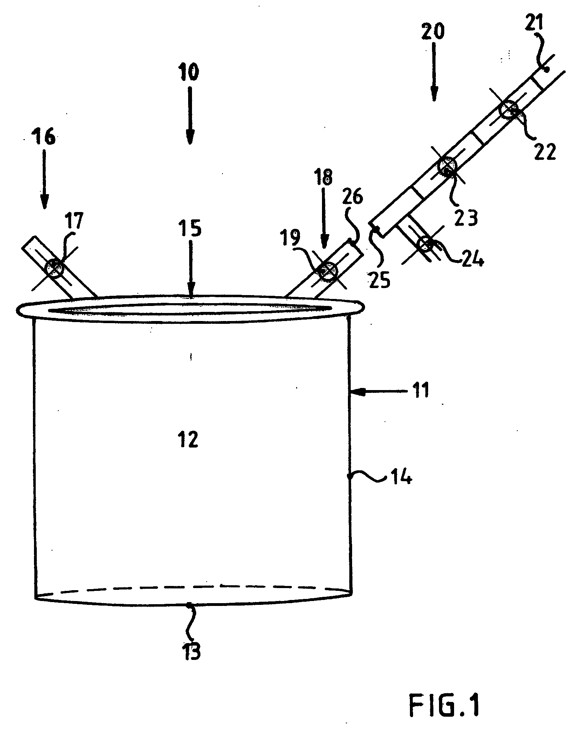

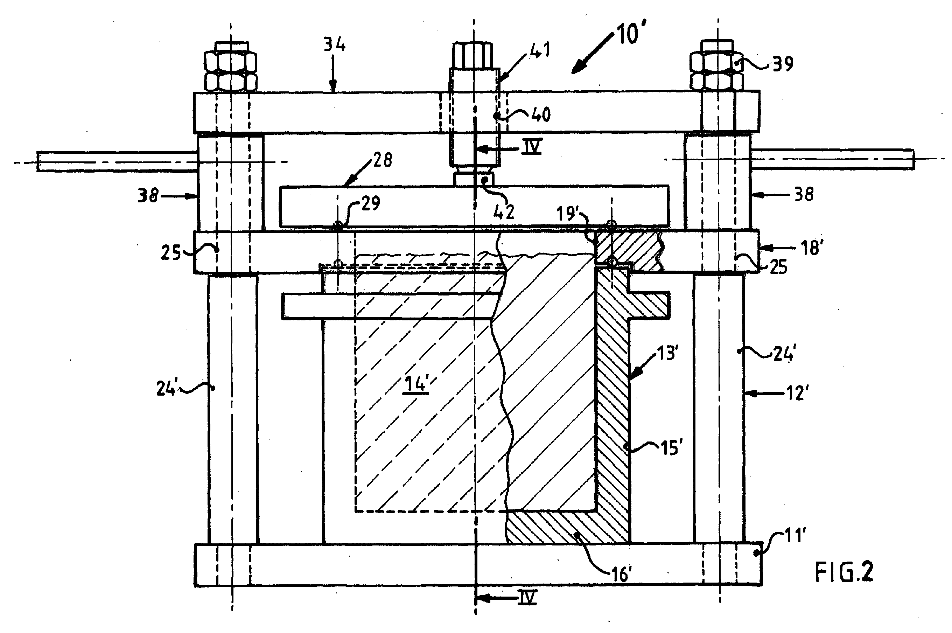

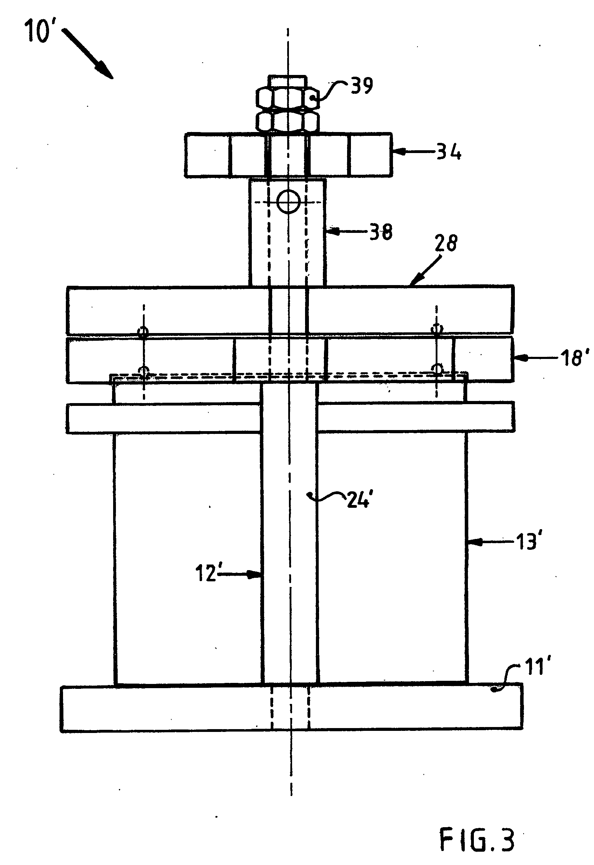

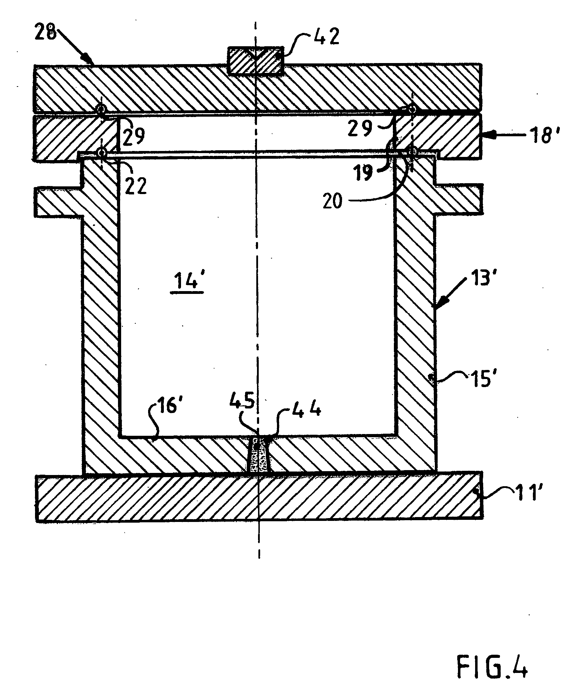

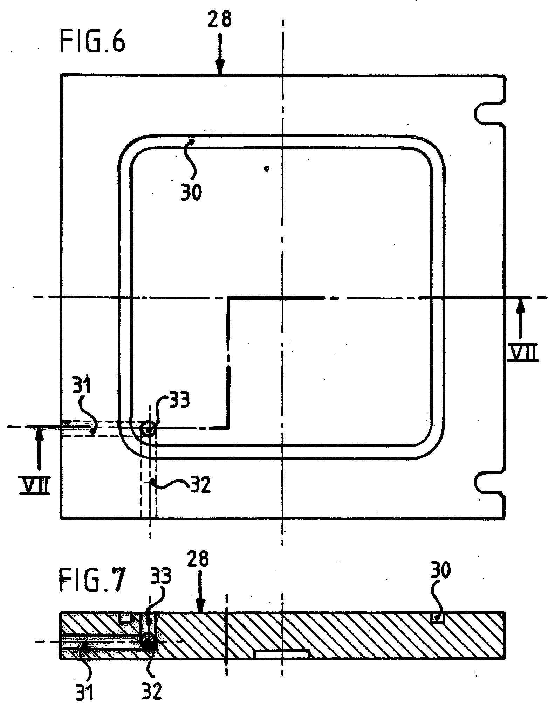

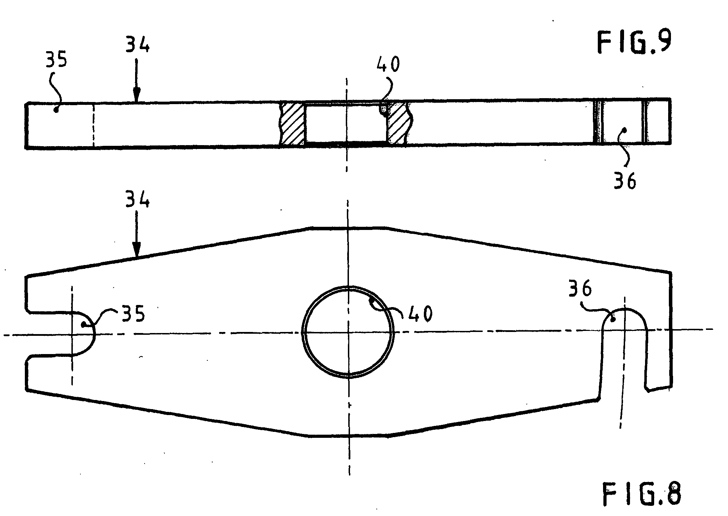

Die in den Figuren 2 bis 9 verwendeten Bezugszeichen sind zur Unterscheidung von den Bezugszeichen in Fig. 1 durch einen Strich gekennzeichnet, soweit Überschneidungen vorliegen.

Bei der zweiten Ausführungsform umfaßt die Vorrichtung 10' einen auf der Grundplatte 11' eines Säulengestells 12' aufstehenden, topfartig ausgebildeten Probenbehälter 13' mit einem Aufnahmeraum 14' für eine Betonprobe. Der Aufnahmeraum 14' weist über seine gesamte Höhe gleichbleibenden Querschnitt auf und ist von einer Behälter-Wandung 15' umgeben sowie unterseitig von einem Behälterboden 16' abgeschlossen. Auf die nach oben weisende Offenseite des Probenbehälters 13' ist ein Rahmen 18' aufgesetzt, der eine mit dem Querschnitt des Aufnahmeraums 14' deckungsgleiche Ausnehmung 19' aufweist. Auf der zum Probenbehälter 13' hinweisenden Seite ist der Rahmen 18' mit einer die Rahmen-Ausnehmung 19' umgebenden Einsenkung 20' versehen, die genau der Außenkontur der Behälter-Wandung 15' angepaßt ist und den oberen Rand der Behälter-Wandung derart übergreift, daß bei aufgesetztem Rahmen 18' dessen Ausnehmung 19' in einer mit dem Querschnitt des Aufnahmeraums 14' deckungsgleichen Lage steht. Im Bereich dieser Einsenkung ist in einer umlaufenden Nut 21' eine O-Ringdichtung 22' aufgenommen, die bei aufgesetztem Rahmen 18' auf der Oberkante der Behälter-Wandung 15' aufsitzt.

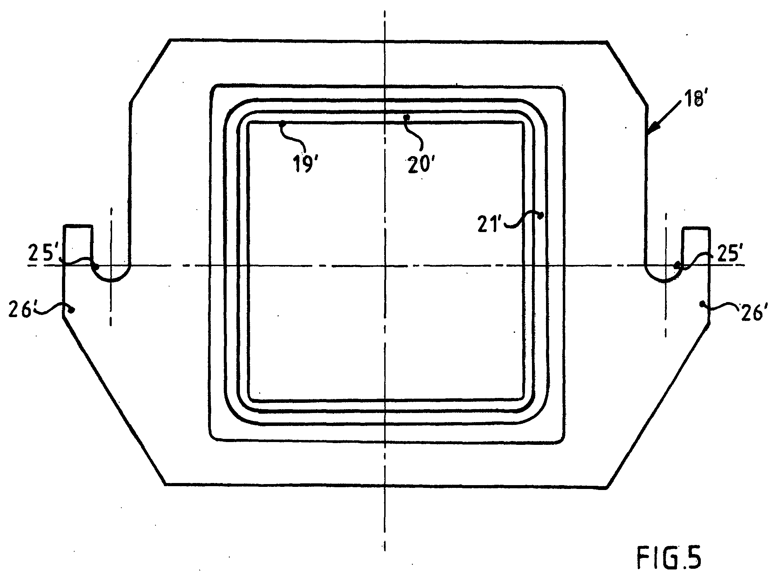

Aufgenommen auf der Grundplatte 11' ist der Probenbehälter 13' zwischen zwei von der Grundplatte 11' nach oben vorstehenden Säulen 24', die ihrerseits seitlich über den Probenbehälter 13' vorstehende und mit entsprechenden Lochungen 25' versehene Abschnitte 26' des Rahmens 18' aufnehmen. Die Lochungen 25' sind, wie Fig. 5 zeigt, als stirnseitig offene Langlöcher ausgebildet, die es ermöglichen, den Rahmen 18' von der Seite her auf die Säulen 24' aufzuschieben.

Gas- und flüssigkeitsdicht abschließbar ist der Aufnahmeraum 14' des Probenbehälters 13' mittels eines oberseitig auf den Rahmen 18' aufsetzbaren Deckels 28', der auf der dem Rahmen zugewandten Seite eine die Rahmen-Ausnehmung 19' umschließende Dichtung 29 besitzt, die in einer umlaufenden Nut 30 des Deckels aufgenommen und ebenfalls als O-Ringdichtung ausgebildet ist. Der Deckel 28 besitzt einen Entlüftungskanal 31, an den ein absperrbares Entlüftungsventil anschließbar ist, und einen Wassereinlaß 32, an den eine Armatur mit einem manuell zwischen einer Schließ- und einer Öffnungslage betätigbaren Einlaßventil anschließbar ist. Der Wassereinlaß 32 und der Entlüftungskanal 31 münden auf der zu dem Rahmen 18' hinweisenden Seite innerhalb des von der O-Ringdichtung 29 des Deckels 28 umschlossenen Fläche bei 33 aus.

Über dem Probenbehälter 13' und beabstandet von dem auf diesem aufgenommenen Rahmen 18' und dem auf letzteren aufsetzbaren Deckel 28 erstreckt sich eine als Quertraverse ausgebildete Spannbrücke 34, die gleichfalls im Bereich ihrer äußeren Enden mit entsprechenden Lochungen 35, 36 versehen und auf den Säulen 24 des Säulengestells 12' aufgenommen sowie dort lagefest positioniert ist. Die Lochungen sind als jeweils einseitig offene Langlöcher ausgebildet und die Lochung 35 erstreckt sich in Längsrichtung der Spannbrücke 34, die Lochung 36 hingegen quer dazu. Der Positionierung der Spannbrücke 34 dienen zwischen dem oberseitig auf dem Probenbehälter 13' aufsitzenden Rahmen 18' und der Spannbrücke 34 sich erstreckende, auf den Säulen 24' aufgenommene Klemmhülsen 38 und auf oberseitige Gewindeabschnitte der Säulen 24' aufgeschraubte Kontermuttern 39, die über die Klemmhülsen 38 eine Verspannung des Rahmens 18' mit dem Probenbehälter 13' bzw. der O-Ringdichtung des Rahmens 18' mit der Oberkante der Behälterwandung 15' vermitteln. Von jeder Klemmhülse 38 erstreckt sich seitlich ein Handhabungsgriff fort.

Im Bereich einer Mittelachse zwischen den Säulen 24' des Säulengestells 12' ist die als Quertraverse ausgebildete Spannbrücke 34 mit einer Spindelmutter bzw. einer entsprechenden Gewindebohrung 40 ausgerüstet, in der eine Gewindespindel 41 mit selbsthemmendem Gewinde aufgenommen ist. Bei auf den Rahmen 18' aufgesetztem Deckel 28 gelingt durch Anziehen der Gewindespindel 41 eine Verspannung des Deckels 28 bzw. der diesem zugeordneten O-Ringdichtung 29 mit dem Rahmen 18', wobei die Gewindespindel 41 über ein Druckstück 42 im Zentrum des Probenbehälters 13' auf den Deckel 28 einwirkt.

Der Aufnahmeraum 14' des Probenbehälters 13' hat quadratischen Querschnitt und eine Höhe, die gleich der Kantenlänge des seinen Querschnitt bildenden Quadrats ist. Die Kantenlänge dieses Quadrats und demgemäß die Höhe des Aufnahmeraums 14' im Probenbehälter 13' ist gleich den Kantenlängen von Probewürfeln für Druckproben bemessen. Diese Kantenlänge beträgt nach den derzeitig gültigen Vorgaben für derartige Probenwürfel in DE 150 mm.

Bei bestimmungsgemäßer Verwendung der erfindungsgemäßen Vorrichtung 10' wird bei abgenommenem Deckel 28 Frischbeton in den Aufnahmeraum 14' des Probenbehälters 13' bis zu einer in die Ausnehmung 19' des auf dem Probenbehälter 13' aufsitzenden Rahmens 18' reichenden Füllhöhe eingebracht und verdichtet. Nach der Bestimmung des Porenanteils der Betonprobe werden der Deckel 28 und der Rahmen 18' abgenommen und der über die Oberkante des Probenbehälters 13' vorstehende Anteil der Betonprobe mittels eines Lineals über die Oberkante des Probenbehälters 13' abgestrichen. Danach kann, sobald die Betonprobe hinreichend abgebunden und damit gestaltfest ist, ein für nachfolgende Druckprüfungen geeigneter Betonwürfel ausgeformt werden.

Um das Ausformen einer abgebundenen Probe aus dem Probenbehälter 13' zu erleichtern, ist der Behälterboden 16' von einer sich zum Aufnahmeraum hin konisch verengenden Ausnehmung 44 durchbrochen, die mittels eines angepaßten, herausnehmbaren Stopfens 45 verschlossen ist. Wenn der Probenbehälter 13' aus dem Säulengestell 12' herausgenommen ist, kann nach dem Entfernen des Stopfens 45 durch die Ausnehmung 44 hindurch die im Aufnahmeraum 14' befindliche Betonprobe beispielsweise mit Druckwasser beaufschlagt und dadurch aus dem Aufnahmeraum 14' des Probenbehälters 13' ausgetrieben werden.

## Patentansprüche

1. Verfahren zum Messen des Luftporenanteils von Frischbeton, bei dem der Frischbeton in einen gas- und flüssigkeitsdicht verschließbaren Probenbehälter eingebracht und verdichtet, dann der Probenbehälter verschlossen und über der Betonprobe vollständig mit Hydraulikmittel blasenfrei aufgefüllt wird,
**dadurch gekennzeichnet, daß**
der Probenbehälter mit der verdichteten Betonprobe vor und nach dem Auffüllen mit Hydraulikmittel jeweils verwogen und das Gewicht der blasenfreien Hydraulikmittelverfüllung durch Differenzbildung der beiden Wiegungen bestimmt wird, daß danach der Probenbehälter an eine Druckmittelquelle angekuppelt und mit unter einer vorbestimmten Spannung stehendem Hydraulikdruckmittel beaufschlagt, dann hermetisch gegenüber der Druckmittelquelle abgesperrt und von dieser abgekuppelt sowie erneut verwogen wird, und daß anschließend als Maß für das Porenvolumen des Frischbetons das Gewicht des infolge der Druckbeaufschlagung in die Betonporen eingedrungenen Hydraulikmittels durch Differenzbildung zwischen der dritten und zweiten Wiegung bestimmt wird.

2. Verfahren nach Anspruch 1, **dadurch gekennzeichnet, daß** die blasenfreie Verfülllung des Probenbehälters über der in diesem aufgenommenen verdichteten Betonprobe bei Atmosphärendruck (etwa 1,0 Bar) erfolgt.

3. Verfahren nach Anspruch 1 oder 2, **dadurch gekennzeichnet, daß** nach dem zweiten Verwiegen des Probenbehälters das in diesem über der Betonprobe aufgenommenen Hydraulikmittel mit unter einem Druck von etwa 3,5 Bar stehenden Hydraulikmittel beaufschlagt wird.

4. Verfahren nach einem der Ansprüche 1 bis 3, **dadurch gekennzeichnet, daß** als Hydraulikmittel zum Auffüllen des Probenbehälters über der Betonprobe und zur nachfolgenden Druckbeaufschlagung Wasser eingesetzt wird.

5. Vorrichtung zum Messen des Luftporenanteils von Frischbeton, die einen topfartig ausgebildeten Probenbehälter mit einem nach oben offenen, über die Höhe gleichbleibenden Querschnitt aufweisenden sowie mittels eines Deckels gas- und flüssigkeitsdicht verschließbaren Aufnahmeraum für eine Betonprobe besitzt, der bei gleichzeitiger Entlüftung über wenigstens ein absperrbares Entlüftungsventil mit Wasser oder einer ähnlichen Flüssigkeit befüllbar ist,
**dadurch gekennzeichnet, daß**
an einen Einlaß (18, 32) des Probenbehälters (11, 13'), der mit einem Absperrventil ausgerüstet ist, ein Hydraulikmittelanschluß ankuppelbar und der Probebehälter (11, 13') mit Hydraulikmittel einer definierten Spannung beaufschlagbar ist.

6. Vorrichtung nach Anspruch 1, **dadurch gekennzeichnet, daß** der Hydraulikmittelanschluß als mit einer Druckmittelquelle verbindbare und mittels einer Schnellkupplung an den Einlaß (18, 32) des Probenbehälters (11, 13')ankuppelbare Armatur ausgebildet ist.

7. Vorrichtung nach Anspruch 2, **dadurch gekennzeichnet, daß** die Armatur (20) mit einem Absperrventil (22) sowie einem zwischen letzterem und der Schnellkupplung angeordneten Druckminderventil (23) ausgerüstet ist.

8. Vorrichtung nach Anspruch 2 oder 3, **dadurch gekennzeichnet, daß** die Armatur (20) angrenzend an die Schnellkupplung mit einem Auslaßventil (24) ausgerüstet ist.

9. Vorrichtung nach einem der Ansprüche 1 bis 4, **dadurch gekennzeichnet, daß** der Probenbehälter (11, 13') mittels eines auf diesen aufsetzbaren Deckels (15, 28) verschließbar ist und daß der mit einem Absperrventil ausgerüstete Behälter-Einlaß (18, 32) und das Entlüftungsventil am Deckel (15, 28) angeordnet sind.

10. Vorrichtung nach einem der Ansprüche 5 bis 9, **dadurch gekennzeichnet, daß** stirnendig auf der Offenseite des Probenbehälters (13) ein abnehmbarer Rahmen (18), der eine mit dem Querschnitt des Behälter-Aufnahmeraums (14) deckungsgleiche Ausnehmung (19) aufweist, unter Zwischenlage einer umlaufenden Dichtung (22) aufgenommen sowie auf den Rahmen (18) der Deckel (28) mit einer sich um die Ausnehmung (19) des Rahmens (18) herumerstreckenden Dichtung (29) und einem auf der zum Rahmen (18) hinweisenden Seite innerhalb der von der Dichtung (29) umschlossenen Fläche ausmündenden, mit einem absperrbaren Ventil versehenen Einlaß (32) für Hydraulikmittel aufsetzbar und mittels einer lösbaren Spannvorrichtung (40, 41, 42) mit dem auf dem Rand des Probenbehälters (13) aufsitzenden Rahmen (18) verspannbar ist.

11. Vorrichtung nach Anspruch 10, **dadurch gekennzeichnet, daß** der Rahmen (18) auf der zum Probenbehälter (13) hinweisenden Seite eine seine Ausnehmung (19) umgebende Einsenkung (20) aufweist, die der Außenkontur des Probenbehälters (13) im Bereich der Behälter-Offenseite angepaßt ist und in die der obere Rand der Behälterwand (15) eingreift.

12. Vorrichtung nach Anspruch 11, **dadurch gekennzeichnet, daß** die mit der Oberkante der Behälterwand (15) zusammenwirkende Dichtung des auf der Behälter-Offenseite aufsitzenden Rahmens (18) als in einer im Bereich der Einsenkung (20) umlaufenden Nut aufgenommene O-Ringdichtung (22) ausgebildet ist.

13. Vorrichtung nach einem der Ansprüche 10 bis 12, **dadurch gekennzeichnet, daß** es sich bei der zwischen dem Deckel (28) und dem Rahmen (18) wirkenden Dichtung um eine in einer im Deckel (28) umlaufenden Nut aufgenommene O-Ringdichtung (29) handelt.

14. Vorrichtung nach einem der Ansprüche 10 bis 13, **dadurch gekennzeichnet, daß** die Spanneinrichtung zum Verspannen des Deckels (28) mit dem Rahmen (18) eine in einer Spindelmutter bzw. einer Gewindebohrung (40) geführte Gewindespindel (41) umfaßt, die zentrisch am Deckel (28) angreift.

15. Vorrichtung nach Anspruch 14, **dadurch gekennzeichnet, daß** die Gewindespindel (41) über ein im Zentrum des Deckels (28) angeordnetes Druckstück (42) auf den Dekkel (28) wirkt.

16. Vorrichtung nach einem der Ansprüche 10 bis 15, **dadurch gekennzeichnet, daß** der Probenbehälter (13) auf einer Grundplatte (11) eines Säulengestells (12) zwischen wenigstens zwei von der Grundplatte (11) nach oben vorstehenden Säulen (24) aufgenommen ist und daß sich oberseitig vom Probenbehälter (13) beabstandet von diesem eine an den Säulen (24) festgelegte Spannbrücke (34) mit der die Gewindespindel (41) führenden Gewindebohrung (40) bzw. einer Spindelmutter erstreckt.

17. Vorrichtung nach Anspruch 16, **dadurch gekennzeichnet, daß** der auf der Offenseite des Probenbehälters (13) aufsitzende Rahmen (18) mit seitlich vom Probenbehälter (13) vorstehenden, mit Lochungen (25) versehenen Abschnitten (26) auf den Säulen (24) des Säulengestells (12) aufgenommen und mit dem Probenbehälter (13) verspannt ist.

18. Vorrichtung nach Anspruch 17, **dadurch gekennzeichnet, daß** die Lochungen (25) in den vorstehenden Abschnitten (26) des Rahmens (18) als einseitig offene Langlöcher ausgebildet sind.

19. Vorrichtung nach Anspruch 17, **dadurch gekennzeichnet, daß** der Rahmen (18) mittels zwischen diesem und der Spannbrücke (34) auf den Säulen (24) aufgenommener Klemmhülsen (38) mit dem Probenbehälter (13) verspannt ist.

20. Vorrichtung nach einem der Ansprüche 16 bis 19, **dadurch gekennzeichnet, daß** die Spannbrücke (34) an beiden Enden mit einseitig offenen Langlöchern (35, 36) versehen und letztere auf die Säulen (24) des Säulengestells (12) aufschiebbar sind.

21. Vorrichtung nach Anspruch 20, **dadurch gekennzeichnet, daß** sich das Langloch (35) an einem Ende der Spannbrükke (34) in deren Längsrichtung erstreckt, hingegen das Langloch (36) am anderen Ende der Spannbrücke quer zu dieser.

22. Vorrichtung nach einem der Ansprüche 10 bis 18, **dadurch gekennzeichnet, daß** der Aufnahmeraum des Probenbehälters quadratisch ausgebildet ist und eine Höhe gleich der Kantenlänge des seinen Querschnitt bildenden Quadrats aufweist.

23. Vorrichtung nach einem der Ansprüche 10 bis 19, **dadurch gekennzeichnet, daß** ein den Aufnahmeraum unterseitig abschließender Behälterboden von wenigstens einer mittels eines entfernbaren Verschlußorgans verschlossenen Ausnehmung durchbrochen ist.

24. Vorrichtung nach Anspruch 20, **dadurch gekennzeichnet, daß** die Ausnehmung im Behälterboden sich zum Aufnahmeraum hin konisch verengt und mittels eines konisch angepaßten Stopfens als Verschlußorgan verschlossen ist.
